# EUROPEAN PATENT SPECIFICATION

(11) **EP 4 174 381 B1**
(45) Date of publication and mention of the grant of the patent: **09.12.2026**
(21) Application number: 22201036.5
(22) Date of filing: 12.10.2022
(51) Int. Cl.: F24C 7/08, G01N 33/00, G01N 33/02

(54) **OVERCOOKING DETECTION METHOD AND HOUSEHOLD APPLIANCE**
VERFAHREN ZUR ERKENNUNG VON ÜBERKOCHEN UND HAUSHALTSGERÄT
PROCÉDÉ DE DÉTECTION DE CUISSON EXCESSIVE ET APPAREIL ÉLECTROMÉNAGER

(30) Priority: 01.11.2021 CN 202111282043
(43) Date of publication of application: 03.05.2023
(73) Proprietor: BSH Hausgeräte GmbH, 81739 München (DE)
(72) Inventor: Qu, Dean, Nanjing 210046 (CN); Du, Fei, Nanjing 210046 (CN); Han, Weiwei, Nanjing (CN); Pang, Zhipeng, Nanjing City, Jiangsu (CN); Ju, Wangkou, Nanjing 210046 (CN)

(56) References cited:
- WO-A1-2022/200006
- CN-A- 112 557 604
- US-A1- 2015 290 795
- US-A1- 2017 130 968

## Description

### TECHNICAL FIELD

Embodiments of the present invention relate to the field of household appliance technologies, and in particular, to an overcooking detection method and a household appliance.

### BACKGROUND

Overcooking has always been one of the key concerns of people in the daily cooking process. Overcooking not only greatly degrades the taste of dishes, but also causes safety hazards, such as fire, in serious cases. Since there is no significant physical quantity change except the taste when overcooking occurs, and there is currently no effective solution that can detect the odor accurately, the prior art cannot effectively resolve the problem of overcooking.

WO 2022 / 200 006 A1 refers to a method of adjusting an operating status of a cooking device based on a predicted internal temperature of cooked food.

US 2015 / 290 795 A1 refers to a method of sensing and later robotically imitating the movements of a chef in a kitchen.

US 2017 / 130 968 A1 and CN 112 557 604 A both refer to respective methods of determining an overcooking stage using machine learning. The methods determine whether the food currently is in an overcooking stage.

### SUMMARY

An objective of embodiments of the present invention is to provide an improved household appliance and an overcooking detection method.

Therefore, an embodiment of the present invention provides an overcooking detection method, including: obtaining gas data, where the gas data includes concentration information of a plurality of types of to-be-detected gas components produced by cooking; inputting the gas data into a preset machine learning model and obtaining a prediction result, where the preset machine learning model is configured to predict, according to the gas data, a current cooking stage and a probability of being in the cooking stage; and determining, according to the prediction result, whether it is in an overcooking stage currently.

This implementation can greatly improve the recognition accuracy of an overcooking phenomenon, which is beneficial to timely detecting and even preventing overcooking, thereby better resolving the overcooking problem. Specifically, with reference to the characteristic that the gas produced in a cooking process is generally mixed gas, in this implementation, concentrations of a plurality of types of gas components volatilized during cooking are acquired and inputted into a preset machine learning model for prediction, so that the preset machine learning model can perform prediction based on the odor actually generated during cooking, thereby obtaining an overcooking recognition result with a high accuracy.

According to the invention, the obtaining gas data includes: adjusting, during operation of a gas detector, a working temperature of the gas detector, and receiving a plurality of pieces of candidate gas data acquired by the gas detector respectively at a plurality of working temperatures, where the plurality of pieces of candidate gas data associated with the different working temperatures are used to represent concentrations of the different to-be-detected gas components produced by cooking; and generating the gas data based on the plurality of pieces of candidate gas data that are received. Therefore, the working temperature of the gas detector is adjusted within a specific temperature range to enrich the diversity of output data of the gas detector, thereby detecting the plurality of types of gas components.

Optionally, the gas detector includes a volatile organic compound (VOC) sensor. Therefore, utilizing the characteristic that the VOC sensor reacts violently to a specific type of gas at different temperatures, the gas detector used in this implementation can detect a plurality of types of gas components within a specific temperature range.

According to the invention, the adjusting, during operation of a gas detector, a working temperature of the gas detector, includes: adjusting, during the operation of the gas detector, the working temperature of the gas detector according to a candidate value selected from a preset working temperature set, where the preset working temperature set includes a plurality of candidate values of the working temperature. The preset working temperature set is designed properly, so that the gas detector can accurately detect a plurality of types of to-be-detected gas components.

According to the invention, the candidate value selected from the preset working temperature set is selected from the preset working temperature set in random order. Correspondingly, the working temperature of the gas detector fluctuates, so that concentrations of the gas components associated with randomly fluctuating candidate values are obtained.

Optionally, the preset working temperature set is traversed for at least one round during the operation of the gas detector. At least one set of data is obtained through cyclic acquisition, so as to expand the volume of data inputted into the preset machine learning model, which is beneficial to improving the accuracy of the prediction result.

Optionally, the gas data is generated based on all pieces of candidate gas data that are received after the preset working temperature set is traversed for at least one round, so that the comprehensiveness of the gas data can be ensured. Alternatively, a quantity of pieces of candidate gas data for generating the gas data is less than a quantity of the candidate values in the preset working temperature set, so that a response speed of the gas detector is higher, and the gas data can be generated more quickly.

Optionally, the candidate values in the preset working temperature set are determined according to sensitivity of the gas detector to the to-be-detected gas components when the gas detector works at the candidate values. Therefore, the gas detector can detect a specific type of gas component at a working temperature at which the gas detector is most sensitive to such a gas component, thereby ensuring the precision of gas data, which is beneficial to ensuring the accuracy of a final prediction result.

Optionally, a training process of the preset machine learning model includes: obtaining a training set, where the training set includes gas data respectively obtained when at least one set of food is in a plurality of cooking stages, where each set of food includes at least one type of food, and the plurality of cooking stages include at least a normal cooking stage and an overcooking stage; and training the preset machine learning model based on the training set until prediction accuracy of the preset machine learning model reaches a preset threshold. Therefore, the diversity of training samples in the training set allows the preset machine learning model to also perform well in a complex environment, and overcooking can be accurately recognized for different foods and different cooking stages.

Optionally, the preset machine learning model is selected from a plurality of candidate models, and the training process of the preset machine learning model further includes: selecting a candidate model with the highest prediction accuracy from a plurality of candidate models obtained by training as the preset machine learning model, where different candidate models are constructed by different algorithms. Therefore, a candidate model with the best prediction performance is selected as the preset machine learning model for final use, which is beneficial to improving the recognition accuracy of overcooking in an actual application stage.

Optionally, the training set further includes cooking data when each piece of gas data is obtained, where the cooking data includes a cooking temperature and a cooking humidity; and the inputting the gas data into a preset machine learning model and obtaining a prediction result includes: inputting the gas data and the cooking data obtained synchronously with the gas data into the preset machine learning model and obtaining the prediction result. Therefore, the diversity of training samples is further expanded with reference to the cooking data, which is beneficial to optimizing a training effect of the preset machine learning model and improving the prediction accuracy.

Optionally, the preset machine learning model includes: a first group of models including a plurality of first candidate models, where each of the first candidate models is obtained by training based on a single training set, the training set has a one-to-one correspondence with a food type, and the training set includes gas data respectively obtained when food of a corresponding type is in a plurality of cooking stages; and a second group of models including at least one second candidate model, where each of the at least one second candidate model is obtained by training based on a plurality of training sets. Therefore, a plurality of types of candidate models are obtained by training using different training sets or different combinations of the training sets, so that different candidate models are sensitive to odors produced by different types of foods or combinations of different types of foods in various cooking stages. In this way, the appropriate candidate model can be used to perform prediction in any odor occasion in the actual application.

Optionally, the inputting the gas data into a preset machine learning model and obtaining a prediction result includes: inputting the gas data into the first group of models and obtaining a plurality of first prediction results outputted by the first candidate models; inputting, if a probability maximum and a secondary probability maximum in the plurality of first prediction results are similar and belong to different cooking stages, the gas data into the second group of models and obtaining second prediction results; and determining, according to a cooking stage to which a probability maximum in the first prediction results and the second prediction results belongs, whether it is in the overcooking stage currently. In this implementation, a comprehensive data model obtained by training based on a plurality of training sets in the second group of models is used as an auxiliary means. When the prediction confidences of the first group of models obtained by training based on a single training set are low, a final prediction result is comprehensively determined with the assistance of the prediction results of the second group of models, which is beneficial to improving the accuracy of the final prediction result outputted by the preset machine learning model.

Optionally, the overcooking detection method further includes: updating a training set based on the obtained gas data and the prediction result, where the training set includes gas data respectively obtained when at least one set of food is in a plurality of cooking stages; and training the preset machine learning model using the updated training set, to obtain an updated preset machine learning model. Therefore, with the increase of a data volume in an actual use process, the training set is continuously expanded, and further, the preset machine learning model is iteratively updated to further improve the prediction accuracy of the preset machine learning model.

Therefore, an embodiment of the present invention further provides a household appliance, including: a control module, configured to perform the overcooking detection method and send a control instruction when it is detected that it is in an overcooking stage currently; and an adjustment module, configured to adjust a cooking parameter according to the control instruction in response to receiving the control instruction. Therefore, when overcooking is detected based on the overcooking detection method provided in this implementation, the cooking parameter is adjusted proactively to prevent overcooking in time.

Optionally, the household appliance is a cooktop, so that the cooktop can proactively monitor an overcooking phenomenon and timely adjust a cooking parameter such as a heating power, to avoid further aggravation of overcooking, thereby optimizing the user experience. Alternatively, the household appliance is a cooker hood, so that the cooker hood performing the overcooking detection method can detect and obtain accurate gas data while exhausting, thereby guiding the cooktop to adjust the cooking parameter timely.

Optionally, the control module is arranged on the cooker hood and/or the cooktop, and the adjustment module is arranged on the cooktop.

Optionally, the household appliance further includes: a gas detector configured to acquire the gas data, where the gas detector communicates with the control module to send the gas data to the control module.

Optionally, the household appliance further includes: a communication module, where the control module communicates with the gas detector through the communication module, and/or the control module communicates with the adjustment module through the communication module.

### BRIEF DESCRIPTION OF THE DRAWINGS

FIG. 1 is a flowchart of an overcooking detection method according to an embodiment of the present invention;
FIG. 2 is a flowchart of a specific implementation of step S101 in FIG. 1;
FIG. 3 is a flowchart of a training process of a preset machine learning model according to an embodiment of the present invention;
FIG. 4 is a flowchart of a specific implementation of step S102 in FIG. 1; and
FIG. 5 is a schematic diagram of a typical application scenario of a household appliance according to an embodiment of the present invention.

In the accompanying drawings,
1 is a household appliance; 10 is a cooker hood; 11 is a cooktop; 12 is a control module; 13 is an adjustment module; 14 is a gas detector; 15 is a communication module; and 16 is a display interface.

### DETAILED DESCRIPTION

To make the foregoing objectives, features, and advantages of the present invention more apparent and easier to understand, specific embodiments of the present invention are described below in detail with reference to the accompanying drawings.

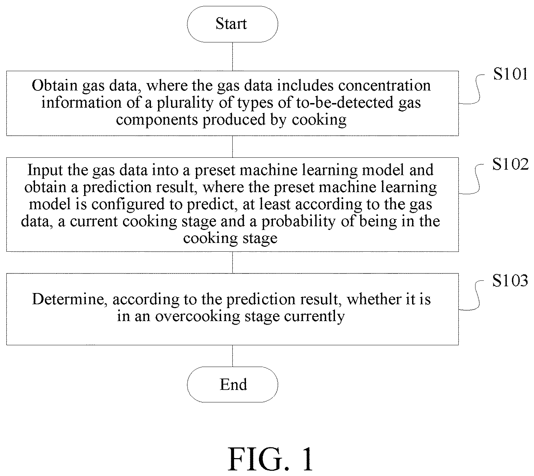

FIG. 1 is a flowchart of an overcooking detection method according to an embodiment of the present invention.

This implementation may be applied to a cooking process monitoring scenario, for example, monitoring a current cooking stage so as to find an overcooking phenomenon timely. In this implementation, a cooking stage during which a single dish is cooked can be detected. For example, in a home cooking scenario, when a user cooks on a cooktop, an odor produced by the cooktop during cooking is detected, to monitor the cooking stage. Alternatively, in this implementation, a whole cooking stage during which a plurality of dishes are cooked at the same time can also be detected. For example, in a catering-kitchen cooking scenario, a mixed odor produced by cooking dishes on a plurality of cooktops can be detected to monitor the whole cooking stage of the plurality of dishes.

Specifically, referring to FIG. 1, the overcooking detection method described in this embodiment may include the following steps:
Step S101: Obtain gas data, where the gas data includes concentration information of a plurality of types of to-be-detected gas components produced by cooking.
Step S102: Input the gas data into a preset machine learning model and obtain a prediction result, where the preset machine learning model is configured to predict, at least according to the gas data, a current cooking stage and a probability of being in the cooking stage.
Step S103: Determine, according to the prediction result, whether it is in an overcooking stage currently.

Further, the to-be-detected gas components may be produced from cooked food. When being cooked, different types of foods may produce different types of gas components, and a same type of food may also produce different types of gas components at different cooking stages. For example, the food may include meat, nuts, vegetables, and the like by type.

Further, the cooking stage may include a normal cooking stage, an overcooking stage, and the like, where the overcooking stage may be further subdivided into a first-level overcooking stage and a second-level overcooking stage, or a mild overcooking stage, a moderate overcooking stage, and a severe overcooking stage. Different types of gas components may be produced at different cooking stages, and the intuitive feeling of a user is that different odors are smelled.

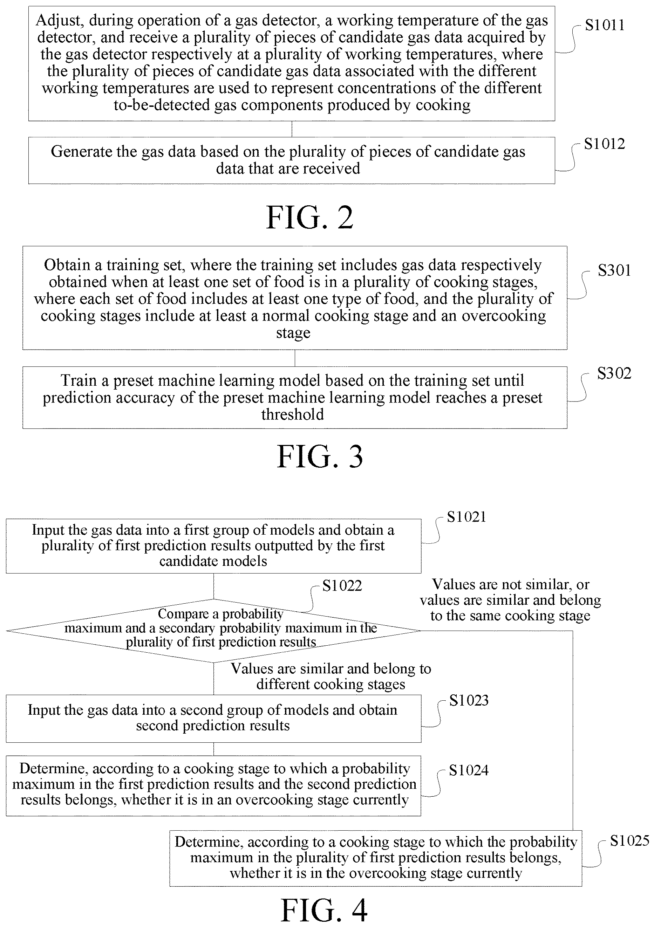

In the invention, with reference to FIG. 2, step S101 includes the following steps:
Step S1011: Adjust, during operation of a gas detector, a working temperature of the gas detector, and receive a plurality of pieces of candidate gas data acquired by the gas detector respectively at a plurality of working temperatures, where the plurality of pieces of candidate gas data associated with the different working temperatures are used to represent concentrations of the different to-be-detected gas components produced by cooking; and
Step S1012: Generate the gas data based on the plurality of pieces of candidate gas data that are received.

Therefore, the working temperature of the gas detector is adjusted within a specific temperature range to enrich the diversity of output data of the gas detector, thereby detecting the plurality of types of gas components.

Specifically, the gas detector may include a VOC sensor. Therefore, utilizing the characteristic that the VOC sensor reacts violently to a specific type of gas at different temperatures, the gas detector used in this implementation can detect a plurality of types of gas components within a specific temperature range. For example, at a specific working temperature, the VOC sensor reacts with a specific type of gas component in the air and outputs a resistance value. The resistance value can be used to represent a concentration of the specific type of gas component.

Further, step S1011 includes the following step: adjusting, during the operation of the gas detector, the working temperature of the gas detector according to a candidate value selected from a preset working temperature set, where the preset working temperature set includes a plurality of candidate values of the working temperature. The preset working temperature set is designed properly, so that the gas detector can accurately detect a plurality of types of to-be-detected gas components.

The candidate values in the preset working temperature set are determined according to sensitivity of the gas detector to the to-be-detected gas components when the gas detector works at the candidate values. For example, a main gas component produced by various foods during overcooking can be determined through pre-experiments. With the gas component as a target, a working temperature of the VOC sensor can be adjusted to test out a working temperature at which the VOC sensor has the highest sensitivity to such a gas component and a time required to detect the gas component. A working temperature at which the sensitivity is the highest is added into the preset working temperature set as a candidate value that can be selected when this implementation is performed. Therefore, the gas detector can detect a specific type of gas component at a working temperature at which the gas detector is most sensitive to such a gas component, thereby ensuring the precision of gas data, which is beneficial to ensuring the accuracy of a final prediction result.

Further, the candidate value selected from the preset working temperature set is selected from the preset working temperature set in random order according to the invention, or in ascending or descending order not according to the invention. Correspondingly, the working temperature of the gas detector continuously fluctuates, rises or falls, so that concentrations of the gas components associated with candidate values are obtained.

For example, when step S1011 is performed, a candidate value with the smallest value may be selected from the preset working temperature set as an initial working temperature of the VOC sensor. After candidate gas data is acquired at the initial working temperature, the working temperature of the VOC sensor is gradually increased until the candidate gas data is acquired under a candidate value with the largest value in the preset working temperature set.

When the VOC sensor performs gas detection in a way of continuous heating or continuous cooling, the working temperature of the VOC sensor does not fluctuate violently in a short time, which is beneficial to prolonging a service life of a device. According to the invention , the VOC sensor selects a candidate value from the preset working temperature set randomly, there is a greater probability to quickly switch to a working temperature at which the VOC sensor is sensitive to a gas component produced by currently cooked food, which is beneficial to a rapid response of the VOC sensor.

Further, the preset working temperature set is traversed for at least one round during the operation of the gas detector. At least one set of data is obtained by cyclic acquisition, so as to expand the volume of data inputted into the preset machine learning model, which is beneficial to improving the accuracy of the prediction result. For example, when step S1011 is performed, the preset working temperature set is repeatedly traversed to obtain concentrations of a plurality of groups of different to-be-detected gas components, and a plurality of concentrations of a same type of gas component are averaged, thereby integrating a plurality of sets of data into a set of gas data and inputting the set of gas data into the preset machine learning model. Therefore, the impact of a single detection error on the prediction result is eliminated by obtaining an average through a plurality of detections.

Further, a time interval during which the VOC sensor switches from a current working temperature to a next working temperature may be determined according to a time required to detect a corresponding gas component at the current working temperature. The required time may be preset through experiments.

Further, the gas data is generated based on all pieces of candidate gas data that are received after the preset working temperature set is traversed for at least one round, so that the comprehensiveness of the gas data can be ensured, and the gas data includes concentration information of all gas components that may be present in the cooking process. For example, when step S1012 is performed, after the preset working temperature set is traversed for one round, all the obtained pieces of candidate gas data are inputted into the preset machine learning model as gas data. In another example, after the preset working temperature set is traversed for a plurality of rounds, a plurality of pieces of candidate gas data obtained at the same working temperature are integrated as a single piece of candidate gas data, and the integrated candidate gas data at all working temperatures is inputted into the preset machine learning model as final gas data.

Alternatively, a quantity of pieces of candidate gas data for generating the gas data may be less than a quantity of the candidate values in the preset working temperature set, so that a response speed of the gas detector is higher, and the gas data can be generated more quickly. For example, when step S1012 is performed, after being obtained, one or more pieces of candidate gas data are inputted into the preset machine learning model as the gas data, so as to obtain a prediction result more quickly.

This implementation can greatly improve the recognition accuracy of an overcooking phenomenon, which is beneficial to timely detecting and even preventing overcooking, thereby better resolving the overcooking problem. Specifically, with reference to the characteristic that the gas produced in a cooking process is generally mixed gas, in this implementation, concentrations of a plurality of types of gas components volatilized during cooking are acquired and inputted into a preset machine learning model for prediction, so that the preset machine learning model can perform prediction based on the odor actually generated during cooking, thereby obtaining an overcooking recognition result with a high accuracy.

In a specific implementation, the candidate values in the preset working temperature set may be used to detect a gas component that may be produced when a variety of foods are cooked. For example, a gas produced when a variety of foods, such as meat for cooking and vegetables, are cooked separately or together is acquired, and gas components are analyzed. Appropriate candidate values are respectively determined according to the various gas components obtained through analysis, to finally generate the preset working temperature set. Correspondingly, when step S1011 is performed, regardless of the type of food currently cooked, a candidate value is selected from a single preset working temperature set as the working temperature of the VOC sensor.

In a variant, the preset working temperature sets can be designed specifically for different types of foods. For example, a preset working temperature set may be constructed for a working temperature at which the VOC sensor is sensitive to a gas component produced during cooking of meat, and a preset working temperature set may be additionally constructed for a working temperature at which the VOC sensor is sensitive to a gas component produced during cooking of nuts. Correspondingly, when step S1011 is performed, a corresponding preset working temperature set is retrieved according to the type of the food currently cooked, and a candidate value is selected from the preset working temperature set as the working temperature of the VOC sensor. When a plurality of types of foods are mixed for cooking, preset working temperature sets corresponding to various foods are respectively retrieved for detection. For example, the types of foods currently cooked may be obtained from information entered by the user, or may be proactively detected and determined by using a sensing device such as an image sensor.

In a specific implementation, with reference to FIG. 3, a training process of the preset machine learning model according to this embodiment may include the following steps:
Step S301: Obtain a training set, where the training set includes gas data respectively obtained when at least one set of food is in a plurality of cooking stages, where each set of food includes at least one type of food, and the plurality of cooking stages include at least a normal cooking stage and an overcooking stage.
Step S302: Train the preset machine learning model based on the training set until prediction accuracy of the preset machine learning model reaches a preset threshold.

Therefore, the diversity of training samples in the training set allows the preset machine learning model to also perform well in a complex environment, and overcooking can be accurately recognized for different foods and different cooking stages.

Specifically, among a plurality of groups of foods included in the training set, some groups may include a single type of food, while some groups may include mixed types of foods, thereby enriching the diversity of the training set.

Further, the preset machine learning model may be selected from a plurality of candidate models, where different candidate models are constructed by using different algorithms.

For example, in step S302, the candidate models may be trained respectively based on the training set until the prediction accuracy of the candidate models reaches a preset threshold. Then, a candidate model with the highest prediction accuracy in a plurality of candidate models obtained by training is selected as the preset machine learning model. Therefore, a candidate model with the best prediction performance is selected as the preset machine learning model for final use, which is beneficial to improving the recognition accuracy of overcooking in an actual application stage.

In another example, in step S302, the candidate models may be trained respectively based on the training set, and a candidate model of which prediction accuracy reaches the preset threshold first in the candidate models is determined as the preset machine learning model.

A specific value of the preset threshold can be set according to the needs of the user. For example, the value may range from 85% to 99%. The preset threshold can be used to measure a tolerance of the user for a prediction error rate of a model, and preset thresholds of different candidate models may be the same or different.

The algorithm used to construct a candidate model may be selected from: multilayer perceptron (MLP), a random forest algorithm, a logistic regression algorithm, a decision tree, an extreme gradient boosting decision tree (XGBoost), a k-nearest neighbor (KNN) classification algorithm, and the like.

The prediction accuracy of the candidate model may be obtained by testing the model with a test set after the model is preliminarily trained based on the training set. For a process of obtaining the test set, reference may be made to a generation process of the training set. The difference is that the test set is not inputted to the candidate model in the model construction stage.

In a typical application scenario, as many types of different foods as possible can be consciously overcooked, some foods that are often cooked together are mixed, and the gas produced during cooking can be acquired. Then, a gas analyzer is configured to analyze a gas component in the acquired gas, for example, analyze a main gas component that appears in the gas during overcooking. A dynamic heating temperature of the VOC sensor is adjusted according to each analyzed gas component (that is, a to-be-detected gas component) produced during overcooking, so that the VOC sensor is relatively sensitive to the to-be-detected gas component. An adjusted VOC sensor is mounted at a proper position of the cooktop and is prepared to acquire overcooking data.

Next, a list of foods is designed for overcooking experiments. The overcooking experiments are carried out under the cooktop, and gas data is acquired during cooking based on the VOC sensor. In addition, calibration personnel are arranged to perform real-time calibration on site. For example, when there is no odor produced due to overcooking, the calibration personnel can calibrate a current stage as a normal cooking stage. When an odor produced due to overcooking occurs for the first time, the calibration personnel can calibrate that overcooking occurs.

Further, the overcooking stage can be further subdivided into a mild burnt smell stage, a moderate burnt smell stage, and a severe burnt smell stage. A training set can be generated according to a real-time calibration result of the calibration personnel and the corresponding gas data.

All the overcooking experiments are performed at least three times, and data is acquired and calibrated during each experiment.

According to the acquired data, data of the last experiment is used as the test set, and the remaining data is divided into a training set and a validation set for generating the preset machine learning model. For example, the preset machine learning model using a specific algorithm is constructed based on the training set. Then, a model parameter of the preset machine learning model is verified and adjusted by using the validation set. Finally, the prediction accuracy of the preset machine learning model is tested by using the test set.

In a specific implementation, the preset machine learning model can predict a cooking stage and a corresponding probability with reference to the gas data and the cooking data. Correspondingly, the training set may further include cooking data when each piece of gas data is obtained, where the cooking data may include a cooking temperature and a cooking humidity.

For example, a temperature and humidity acquisition device may be arranged close to a cooktop, or a temperature and humidity acquisition device may be arranged on a cooker hood to acquire temperature and humidity data during cooking. In another example, the cooking data can be acquired by multiplexing the VOC sensor.

Correspondingly, step S102 may include the following step: inputting the gas data and the cooking data obtained synchronously with the gas data into the preset machine learning model and obtaining the prediction result. Therefore, the diversity of training samples is further expanded with reference to the cooking data, which is beneficial to optimizing a training effect of the preset machine learning model and improving the prediction accuracy.

In a specific implementation, the preset machine learning model may include a first group of models and a second group of models. The first group of models may include a plurality of first candidate models, where each of the first candidate models is obtained by training based on a single training set, the training set has a one-to-one correspondence with a food type, and the training set includes gas data respectively obtained when food of a corresponding type is in a plurality of cooking stages. The second group of models may include at least one second candidate model, where each of the at least one second candidate model is obtained by training based on a plurality of training sets.

Specifically, the first candidate models may be constructed by using the same or different algorithms. Similarly, the second candidate model may be constructed by using an algorithm the same or different from that of the first candidate model, or a plurality of second candidate models may also be constructed by using the same or different algorithms

Further, for any first candidate model or second candidate model, a candidate model with the best prediction performance may be selected from a plurality of candidate models constructed based on the same training set and different algorithms.

For example, the second candidate model may be obtained by training based on all the training sets. In another example, it is assumed that there are three training sets: a training set 1, a training set 2, and a training set 3. The second group of models may include two second candidate models in total: a second candidate model 1 and a second candidate model 2, where the second candidate model 1 may be obtained by training based on the training set 1 and the training set 2, and the second candidate model 2 may be obtained by training based on the training set 1, the training set 2, and the training set 3.

That is, the first candidate model can be obtained by training based on gas data during cooking of a single type of food, and the second candidate model can be obtained by training based on gas data during cooking of mixed types of foods. Gas data associated with mixed types of foods can be regarded as complex data, and the gas data associated with a single type of food can be regarded as simple data. The second candidate model constructed based on the complex data has high adaptability and can reliably predict cooking stages of the plurality of types of foods in the actual application. The first candidate model constructed based on simple data has high accuracy in predicting a cooking stage of a specific type of food. For example, if the first candidate model is obtained by training based on a training set generated during cooking of meat, the first candidate model performs well in predicting the cooking stage of meat in subsequent use.

Therefore, a plurality of types of candidate models are obtained by training using different training sets or different combinations of the training sets, so that different candidate models are sensitive to odors produced by different types of foods or combinations of different types of foods in various cooking stages. In this way, the appropriate candidate model can be used to perform prediction in any odor occasion in the actual application.

Further, referring to FIG. 4, step S102 may include the following steps:
Step S1021: Input the gas data into the first group of models and obtain a plurality of first prediction results outputted by the first candidate models.
Step S1022: Compare a probability maximum and a secondary probability maximum in the plurality of first prediction results;

If values (that is, the probability maximum and the secondary probability maximum in the plurality of first prediction results) are similar and belong to different cooking stages, step S1023 and step S1024 are performed. Step S1023 includes: inputting the gas data into the second group of models and obtaining second prediction results. Step S1024 includes: determining, according to a cooking stage to which a probability maximum in the first prediction results and the second prediction results belongs, whether it is in the overcooking stage currently.

If a comparison result in step S1022 is that values (that is, the probability maximum and the secondary probability maximum in the plurality of first prediction results) are not similar, or values are similar and belong to the same cooking stage, step S1025 is performed to determine, according to a cooking stage to which the probability maximum in the plurality of first prediction results belongs, whether it is in the overcooking stage currently.

Specifically, when the probability maximum value and the secondary probability maximum value in the plurality of first prediction results are similar and belong to different cooking stages, it indicates that prediction confidences of the first group of models for the current cooking stage prediction are low, so that a current actual cooking stage is determined with the assistance of the second prediction results of the second group of models.

For example, when a difference between the probability maximum value and the secondary probability maximum value in the first prediction results is less than 10%, the two values can be considered as similar. In the actual application, the difference may be adjusted as required.

According to the above, in this implementation, a comprehensive data model obtained by training based on a plurality of training sets in the second group of models is used as an auxiliary means. When the prediction confidences of the first group of models obtained by training based on a single training set are low, a final prediction result is comprehensively determined with the assistance of the prediction results of the second group of models, which is beneficial to improving the accuracy of the final prediction result outputted by the preset machine learning model.

In a specific implementation, in step S103, if the prediction result outputted by the preset machine learning model is an overcooking stage, it is directly determined that it is in the overcooking stage currently.

Alternatively, if the prediction result is that it is in an overcooking stage currently, whether a probability outputted by the preset machine learning model is higher than a preset standard value is determined. If the probability is higher than the preset standard value, it is determined that it is in the overcooking stage currently. If the probability is lower than the preset standard value, step S 101 and step S 102 are performed again until the prediction probability exceeds the preset standard value. For example, the preset standard value may be 60%. In the actual application, the value may be adjusted as required.

In a specific implementation, after step S103, the overcooking detection method in this implementation may further include the following step: updating a training set based on the obtained gas data and the prediction result, where the training set includes gas data respectively obtained when at least one set of food is in a plurality of cooking stages; training the preset machine learning model using the updated training set, to obtain an updated preset machine learning model. Therefore, with the increase of a data volume in an actual use process, the training set is continuously expanded, and further, the preset machine learning model is iteratively updated to further improve the prediction accuracy of the preset machine learning model.

Further, after the prediction result is obtained in step S102, the prediction result may be sent to the user, and feedback from the user may be received. If the feedback from the user indicates that the current prediction result has a deviation, the training set is updated based on a prediction result corrected by the user to update the preset machine learning model iteratively.

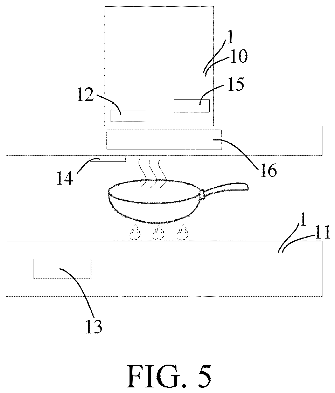

FIG. 5 is a schematic diagram of a typical application scenario of a household appliance according to an embodiment of the present invention.

Specifically, referring to FIG. 5, the household appliance 1 according to this embodiment may include: a control module 12, configured to perform the overcooking detection method shown in FIG. 1 to FIG. 4 and send a control instruction when it is detected that it is in an overcooking stage currently; an adjustment module 13, configured to adjust a cooking parameter according to the control instruction in response to receiving the control instruction.

For example, the household appliance 1 may include a kitchen appliance such as a cooktop 11, a cooker hood 10, and the like.

Therefore, when overcooking is detected based on the overcooking detection method provided in this implementation, the cooking parameter is adjusted proactively to prevent overcooking in time.

For example, the control module 12 may be arranged on the cooker hood 10, and the adjustment module 13 may be arranged on the cooktop 11. For example, the control module 12 may be a main control board of the cooker hood 10. For example, the adjustment module 13 may be a main control board of the cooktop 11, where the main control board of the cooktop 11 may be used to adjust a heating power, a fire power, and the like of the cooktop 11.

Further, the household appliance 1 may further include a gas detector 14, configured to acquire the gas data, where the gas detector 14 communicates with the control module 12 to send the gas data to the control module 12. For example, the gas detector 14 may be arranged on the cooker hood 10, to detect and obtain the gas data when the cooker hood 10 exhausts. Further, a surface of the gas detector 14 may be covered with a protective film to reduce impact of moisture produced during cooking on the detection accuracy.

Communication between the gas detector 14 and the control module 12 and between the control module 12 and the adjustment module 13 may be performed in a wired or wireless manner.

Further, the household appliance 1 may further include a communication module 15, where the control module 12 communicates with the gas detector 14 through the communication module 15. For example, the communication module 15 may be arranged on the cooker hood 10, to perform signal transmission between the control module 12 and the gas detector 14.

Further, the control module 12 may also communicate with the adjustment module 13 through the communication module 15.

For example, a communication module (not shown in the figure) may be integrated into the cooktop 11, and communicate with the control module 12, to receive the control instruction.

Alternatively, the communication module 15 may communicate with a device terminal of a user to inform the user of a prediction result in time. For example, the device terminal may include a mobile phone, an iPAD, a home monitoring device, and the like.

It should be noted that FIG. 5 only exemplarily shows possible arrangement positions of the control module 12, the adjustment module 13, the gas detector 14, and the communication module 15 on the household appliance 1. In the actual application, mutual position relationships of modules and specific arrangement positions on the household appliance 1 may be adjusted according to needs. The modules may be independent of each other, or may be integrated on a same chip or integrated into a same functional module. For example, the control module 12 and the communication module 15 may be integrated together.

Therefore, the cooktop 11 can proactively monitor an overcooking phenomenon and timely adjust a cooking parameter such as a heating power, to avoid further aggravation of overcooking, thereby optimizing the user experience. The cooker hood 10 performing the overcooking detection method can detect and obtain an accurate gas data while exhausting, thereby guiding the cooktop 11 to adjust the cooking parameter timely.

In a specific application scenario, the preset machine learning model may be deployed on the control module 12, and the gas detector 14 may be connected to the control module 12 using a specific communication protocol.

A trigger key (not shown in the figure) may be arranged in a display interface 16 of the cooker hood 10, and when clicking the key, the user triggers a function of monitoring overcooking. Alternatively, the user may remotely trigger the function through an intelligent terminal communicating with the cooker hood 10.

The control module 12 performs the overcooking detection method shown in FIG. 1 to FIG. 4 in response to the function being triggered. Specifically, the control module 12 controls operation of the gas detector 14 to acquire gas data during cooking. Further, the gas detector 14 sends the acquired gas data to the control module 12. Then, the control module 12 inputs the gas data into the preset machine learning model and obtains the prediction result. Therefore, whether there is an overcooking event can be monitored in real time.

If the prediction result indicates that it is in an overcooking stage currently, the control module 12 generates a control instruction and sends the control instruction to the adjustment module 13 through the communication module 15. The adjustment module 13 adjusts a cooking parameter according to the control instruction, so as to alleviate or eliminate the overcooking event. For example, the control instruction may be reducing the heating power or stopping heating, to instruct the cooktop 11 to take a measure to reduce further overcooking.

Further, an appropriate control instruction may be generated according to a degree of overcooking indicated by the prediction result. For example, if the prediction result represents that it is in a mild overcooking stage currently, the control instruction may instruct the adjustment module 13 to reduce the heating power. In another example, if the prediction result indicates that it is in a moderate overcooking stage currently, the control instruction may instruct the adjustment module 13 to stop heating directly.

In a variant, the control module 12 may also be arranged on the cooktop 11, so that the gas data and the control instruction are both transmitted in the cooktop 11 without remote interaction with another device.

Although specific implementations are described above, the implementations are not intended to limit the scope disclosed in the present disclosure, even if only a single implementation is described with respect to a specific feature. The feature example provided in the present disclosure is intended to be illustrative rather than limitative, unless otherwise stated. In specific implementations, the technical features of one or more dependent claims may be combined with the technical features of the independent claims, and the technical features from the corresponding independent claims may be combined in any appropriate manner, rather than only in the specific combinations listed in the claims.

Although the present invention is disclosed above, the present invention is not limited thereto. Any person skilled in the art can make various changes and modifications without departing from the scope of the present invention. Therefore, the protection scope of the present invention should be subject to the scope defined by the claims.

## Claims

1. An overcooking detection method comprising:
- obtaining (S101) gas data comprising concentration information of a plurality of types of to-be-detected gas components produced by cooking; including
- adjusting (S1011), during operation of a gas detector, a working temperature of the gas detector according to a candidate value selected from a preset working temperature set, wherein the preset working temperature set comprises a plurality of candidate values of the working temperature,
- receiving a plurality of pieces of candidate gas data acquired by the gas detector respectively at a plurality of working temperatures, wherein the plurality of pieces of candidate gas data associated with the different working temperatures are used to represent concentrations of the different to-be-detected gas components produced by cooking; and
- generating (S1012) the gas data based on the plurality of pieces of candidate gas data that are received;
- inputting (S102) the gas data into a preset machine learning model and obtaining a prediction result, wherein the preset machine learning model is configured to predict, at least according to the gas data, a current cooking stage and a probability of being in the cooking stage; and
- determining (S103), according to the prediction result, whether it is in an overcooking stage currently;
wherein the candidate value is selected from the preset working temperature set in random order.

2. The overcooking detection method according to claim 1, **characterized in that** the gas detector comprises a volatile organic compound (VOC) sensor.

3. The overcooking detection method according to claim 2, **characterized in that** the candidate value is selected from a single preset working temperature set as the working temperature of the volatile organic compound (VOC) sensor.

4. The overcooking detection method according to any of claims 1 to 3,
further comprising:
- sending, by a control module (12) of a household appliance (1), a control instruction when it is detected that it is in an overcooking stage currently; and
- adjusting, by an adjustment module (13) of the household appliance (1), a cooking parameter according to the control instruction in response to receiving the control instruction by an adjustment module (13).

5. The overcooking detection method according to any of the preceeding claims,
**characterized in that** the preset working temperature set is traversed for at least one round during the operation of the gas detector.

6. The overcooking detection method according to any of the preceeding claims,
**characterized in that** the gas data is generated based on all pieces of candidate gas data that are received after the preset working temperature set is traversed for at least one round, or a quantity of pieces of candidate gas data for generating the gas data is less than a quantity of the candidate values in the preset working temperature set.

7. The overcooking detection method according to any of the preceeding claims,
**characterized in that** the candidate values in the preset working temperature set are determined according to sensitivity of the gas detector to the to-be-detected gas components when the gas detector works at the candidate values.

8. The overcooking detection method according to any of the preceeding claims,
**characterized in that** a training process of the preset machine learning model comprises:
- obtaining (S301) a training set, wherein the training set comprises gas data respectively obtained when at least one set of food is in a plurality of cooking stages, wherein each set of food comprises at least one type of food, and the plurality of cooking stages comprise at least a normal cooking stage and an overcooking stage; and
- training (S302) the preset machine learning model based on the training set until prediction accuracy of the preset machine learning model reaches a preset threshold.

9. The overcooking detection method according to claim 8, **characterized in that** the preset machine learning model is selected from a plurality of candidate models, and
that the training process of the preset machine learning model selected from the plurality of candidate models further comprises:
- selecting, from a plurality of candidate models obtained by training as the preset machine learning model, with different candidate models being constructed by different algorithms, a candidate model with the highest prediction accuracy as the preset machine learning model.

10. The overcooking detection method according to any of the preceeding claims,
**characterized in that** the training set further comprises cooking data when each piece of gas data is obtained, wherein the cooking data comprises a cooking temperature and a cooking humidity; and the inputting the gas data into a preset machine learning model and obtaining a prediction result comprises: inputting the gas data and the cooking data obtained synchronously with the gas data into the preset machine learning model and obtaining the prediction result.

11. The overcooking detection method according to any of the preceeding claims,
**characterized in that** the preset machine learning model comprises:
- a first group of models comprising a plurality of first candidate models, wherein each of the first candidate models is obtained by training based on a single training set, the training set has a one-to-one correspondence with a food type, and the training set comprises gas data respectively obtained when food of a corresponding type is in a plurality of cooking stages; and
- a second group of models comprising at least one second candidate model, wherein each of the at least one second candidate model is obtained by training based on a plurality of training sets.

12. The overcooking detection method according to any of the preceeding claims,
**characterized in that** the inputting the gas data into a preset machine learning model and obtaining a prediction result comprises:
- inputting (S1021) the gas data into the first group of models and obtaining (S1021) a plurality of first prediction results outputted by the first candidate models;
- inputting (S1023), if a probability maximum and a secondary probability maximum in the plurality of first prediction results are similar and belong to different cooking stages, the gas data into the second group of models and obtaining (S1023) second prediction results; and
- determining (S1024), according to a cooking stage to which a probability maximum in the first prediction results and the second prediction results belongs, whether it is in the overcooking stage currently.

13. The overcooking detection method according to any of the preceeding claims,
**characterized by** further comprising:
- updating a training set based on the obtained gas data and the prediction result, wherein the training set comprises gas data respectively obtained when at least one set of food is in a plurality of cooking stages; and
- training the preset machine learning model using the updated training set, to obtain an updated preset machine learning model.

14. A household appliance (1), **characterized by** comprising:
- a control module (12), configured to perform the overcooking detection method according to any one of claims 1 to 13, and to send a control instruction when it is detected that it is in an overcooking stage currently; and
- an adjustment module (13), configured to adjust a cooking parameter according to the control instruction in response to receiving the control instruction.

15. The household appliance (1) according to claim 14, **characterized in that** the household appliance (1) further comprises at least one of a cooktop (11) or a cooker hood (10).

16. The household appliance (1) according to claim 14, **characterized by** further comprising: a gas detector (14) configured to acquire the gas data, wherein the gas detector (14) communicates with the control module (12) to send the gas data to the control module (12).

17. The household appliance (1) according to any of claims 14 to 16, **characterized by** further comprising: a communication module (15), wherein the control module (12) communicates with the gas detector (14) through the communication module (15), and/or the control module (12) communicates with the adjustment module (13) through the communication module (15).

## Patentansprüche

1. Verfahren zum Erkennen eines Verkochens, das Folgendes umfasst:
- Erfassen (S101) von Gasdaten, die Konzentrationsangaben zu mehreren Arten zu erkennender Gasbestandteile umfassen, welche beim Kochen erzeugt werden, einschließlich
- Anpassen (S1011) einer Arbeitstemperatur eines Gasdetektors entsprechend einem aus einem voreingestellten Arbeitstemperatursatz ausgewählten möglichen Wert beim Betrieb des Gasdetektors, wobei der voreingestellte Arbeitstemperatursatz mehrere mögliche Werte für die Arbeitstemperatur umfasst,
- Empfangen mehrerer vom Gasdetektor jeweils bei mehreren Arbeitstemperaturen erfasster möglicher Gasdaten, wobei die mehreren mit den verschiedenen Arbeitstemperaturen verknüpften möglichen Gasdaten zum Darstellen von Konzentrationen der verschiedenen zu erkennenden Gasbestandteile, die beim Kochen erzeugt werden, benutzt werden, und
- Generieren (S1012) der Gasdaten auf der Grundlage der mehreren empfangenen möglichen Gasdaten,
- Eingeben (S102) der Gasdaten in ein voreingestelltes maschinelles Lernmodell und Erhalten eines Prognoseergebnisses, wobei das voreingestellte maschinelle Lernmodell so konfiguriert ist, dass es zumindest entsprechend den Gasdaten einen derzeitigen Garzustand und eine Wahrscheinlichkeit seines Bestehens prognostiziert, und
- entsprechend dem Prognoseergebnis Ermitteln (S103), ob derzeit ein Verkochzustand besteht,
wobei der mögliche Wert in willkürlicher Reihenfolge aus dem voreingestellten Arbeitstemperatursatz ausgewählt wird.

2. Verfahren zum Erkennen eines Verkochens nach Anspruch 1, **dadurch gekennzeichnet, dass** der Gasdetektor einen Sensor für flüchtige organische Verbindungen (VOC-Sensor) umfasst.

3. Verfahren zum Erkennen eines Verkochens nach Anspruch 2, **dadurch gekennzeichnet, dass** der mögliche Wert aus einem einzigen voreingestellten Arbeitstemperatursatz als Arbeitstemperatur des Sensors für flüchtige organische Verbindungen ausgewählt wird.

4. Verfahren zum Erkennen eines Verkochens nach einem der Ansprüche 1 bis 3, das ferner Folgendes umfasst:
- Senden einer Steueranweisung durch ein Steuermodul (12) eines Haushaltsgeräts (1), wenn erkannt wird, dass derzeit ein Verkochzustand besteht, und
- Anpassen eines Garparameters entsprechend der Steueranweisung durch ein Anpassmodul (13) des Haushaltsgeräts (1) als Reaktion auf das Empfangen der Steueranweisung durch ein Anpassungsmodul (13).

5. Verfahren zum Erkennen eines Verkochens nach einem der vorhergehenden Ansprüche, **dadurch gekennzeichnet, dass** der voreingestellte Arbeitstemperatursatz beim Betrieb des Gasdetektors für mindestens eine Runde durchlaufen wird.

6. Verfahren zum Erkennen eines Verkochens nach einem der vorhergehenden Ansprüche, **dadurch gekennzeichnet, dass** die Gasdaten auf der Grundlage sämtlicher möglicher Gasdaten generiert werden, die nach dem Durchlaufen des voreingestellten Arbeitstemperatursatzes für mindestens eine Runde empfangen werden, oder eine Anzahl möglicher Gasdaten zum Generieren der Gasdaten geringer ist als eine Anzahl der möglichen Werte in dem voreingestellten Arbeitstemperatursatz.

7. Verfahren zum Erkennen eines Verkochens nach einem der vorhergehenden Ansprüche, **dadurch gekennzeichnet, dass** die möglichen Werte in dem voreingestellten Arbeitstemperatursatz entsprechend einer Empfindlichkeit des Gasdetektors für die zu erkennenden Gasbestandteile ermittelt werden, wenn der Gasdetektor mit den möglichen Werten arbeitet.

8. Verfahren zum Erkennen eines Verkochens nach einem der vorhergehenden Ansprüche, **dadurch gekennzeichnet, dass** ein Trainingsprozess für das voreingestellte maschinelle Lernmodell Folgendes umfasst:
- Erhalten (S301) eines Trainingssatzes, wobei der Trainingssatz Gasdaten umfasst, die jeweils erfasst werden, wenn sich mindestens ein Satz Lebensmittel in mehreren Garzuständen befindet, wobei jeder Satz Lebensmittel mindestens eine Art Lebensmittel umfasst und die mehreren Garzustände zumindest einen normalen Garzustand und einen Verkochzustand umfassen, und
- Trainieren (S302) des voreingestellten maschinellen Lernmodells auf der Grundlage des Trainingssatzes, bis die Prognosegenauigkeit des voreingestellten maschinellen Lernmodells einen voreingestellten Schwellenwert erreicht.

9. Verfahren zum Erkennen eines Verkochens nach Anspruch 8, **dadurch gekennzeichnet, dass** das voreingestellte maschinelle Lernmodell aus mehreren möglichen Modellen ausgewählt wird und
dass der Trainingsprozess für das voreingestellte maschinelle Lernmodell, das unter den mehreren möglichen Modellen ausgewählt wird, ferner Folgendes umfasst:
- Auswählen eines möglichen Modells mit der höchsten Prognosegenauigkeit aus mehreren möglichen Modellen, die durch Trainieren als voreingestelltes maschinelles Lernmodell erhalten werden, wobei verschiedene mögliche Modelle durch verschiedene Algorithmen konstruiert werden, als voreingestelltes maschinelles Lernmodell.

10. Verfahren zum Erkennen eines Verkochens nach einem der vorhergehenden Ansprüche, **dadurch gekennzeichnet, dass** der Trainingssatz ferner Gardaten umfasst, wenn jedes Gasdatenelement erfasst wird, wobei die Gardaten eine Gartemperatur und eine Garfeuchtigkeit umfassen und das Eingeben der Gasdaten in ein voreingestelltes maschinelles Lernmodell und das Erhalten eines Prognoseergebnisses Folgendes umfasst: Eingeben der Gasdaten und der gleichzeitig mit den Gasdaten erfassten Gardaten in das voreingestellte maschinelle Lernmodell und Erhalten des Prognoseergebnisses.

11. Verfahren zum Erkennen eines Verkochens nach einem der vorhergehenden Ansprüche, **dadurch gekennzeichnet, dass** das voreingestellte maschinelle Lernmodell Folgendes umfasst:
- eine erste Gruppe von Modellen, die mehrere erste mögliche Modelle umfasst, wobei jedes der ersten möglichen Modelle durch Trainieren auf der Grundlage eines einzigen Trainingssatzes erhalten wird, der eindeutig einer Lebensmittelart entspricht und Gasdaten umfasst, die jeweils erfasst werden, wenn sich ein Lebensmittel einer entsprechenden Art in mehreren Garzuständen befindet, und
- eine zweite Gruppe von Modellen, die mindestens ein zweites mögliches Modell umfasst, wobei jedes des mindestens einen zweiten möglichen Modells durch Trainieren auf der Grundlage mehrerer Trainingssätze erhalten wird.

12. Verfahren zum Erkennen eines Verkochens nach einem der vorhergehenden Ansprüche, **dadurch gekennzeichnet, dass** das Eingeben der Gasdaten in ein voreingestelltes maschinelles Lernmodell und das Erhalten eines Prognoseergebnisses Folgendes umfasst:
- Eingeben (S1021) der Gasdaten in die erste Gruppe von Modellen und Erhalten (S1021) mehrerer erster Prognoseergebnisse, die von den ersten möglichen Modellen ausgegeben werden,
- Eingeben (S1023) der Gasdaten in die zweite Gruppe von Modellen und Erhalten (S1023) von zweiten Prognoseergebnissen, wenn sich ein Wahrscheinlichkeitsmaximum und ein sekundäres Wahrscheinlichkeitsmaximum bei den mehreren ersten Prognoseergebnissen gleichen und zu verschiedenen Garzuständen gehören, und
- entsprechend einem Garzustand, zu dem ein Wahrscheinlichkeitsmaximum bei den ersten und den zweiten Prognoseergebnissen gehört, Ermitteln (S1024), ob derzeit ein Verkochzustand besteht.

13. Verfahren zum Erkennen eines Verkochens nach einem der vorhergehenden Ansprüche, **dadurch gekennzeichnet, dass** es ferner Folgendes umfasst:
- Aktualisieren eines Trainingssatzes auf der Grundlage der erfassten Gasdaten und des Prognoseergebnisses, wobei der Trainingssatz Gasdaten umfasst, die jeweils erfasst werden, wenn sich mindestens ein Satz Lebensmittel in mehreren Garzuständen befindet, und
- Trainieren des voreingestellten maschinellen Lernmodells unter Verwendung des aktualisierten Trainingssatzes zwecks Erhaltens eines aktualisierten voreingestellten maschinellen Lernmodells.

14. Haushaltsgerät (1), **dadurch gekennzeichnet, dass** es Folgendes umfasst:
- ein Steuermodul (12), das so konfiguriert ist, dass es das Verfahren zum Erkennen eines Verkochens nach einem der Ansprüche 1 bis 13 durchführt und eine Steueranweisung sendet, wenn ermittelt wird, dass derzeit ein Verkochzustand besteht, und
- ein Anpassungsmodul (13), das so konfiguriert ist, dass es als Reaktion auf das Empfangen der Steueranweisung der Steueranweisung entsprechend einen Garparameter anpasst.

15. Haushaltsgerät (1) nach Anspruch 14, **dadurch gekennzeichnet, dass** das Haushaltsgerät (1) ferner eine Herdplatte (11) oder eine Dunstabzugshaube (10) umfasst.

16. Haushaltsgerät (1) nach Anspruch 14, **dadurch gekennzeichnet, dass** es ferner Folgendes umfasst: einen Gasdetektor (14), der so konfiguriert ist, dass er die Gasdaten erfasst, wobei der Gasdetektor (14) mit dem Steuermodul (12) kommuniziert und so die Gasdaten zu dem Steuermodul (12) sendet.

17. Haushaltsgerät (1) nach einem der Ansprüche 14 bis 16, **dadurch gekennzeichnet, dass** es ferner Folgendes umfasst: ein Kommunikationsmodul (15), wobei das Steuermodul (12) über das Kommunikationsmodul (15) mit dem Gasdetektor (14) und/oder mit dem Anpassungsmodul (13) kommuniziert.

## Revendications

1. Procédé de détection de cuisson excessive, comprenant :
- l'obtention (S101) de données de gaz comprenant des informations de concentration d'une pluralité de types de composants de gaz à détecter produits par cuisson ; comportant
- l'ajustement (S1011), pendant le fonctionnement d'un détecteur de gaz, d'une température de travail du détecteur de gaz en fonction d'une valeur candidate sélectionnée à partir d'un ensemble de températures de travail prédéfini, dans lequel l'ensemble de températures de travail prédéfini comprend une pluralité de valeurs candidates de la température de travail,
- la réception d'une pluralité de données de gaz candidates acquises par le détecteur de gaz, respectivement, à une pluralité de températures de travail, dans lequel la pluralité de données de gaz candidates associées aux différentes températures de travail est utilisée pour représenter des concentrations des différents composants de gaz à détecter produits par cuisson ; et
- la génération (S1012) des données de gaz sur la base de la pluralité de données de gaz candidates qui sont reçues ;
- l'entrée (S102) des données de gaz dans un modèle d'apprentissage automatique prédéfini et l'obtention d'un résultat de prédiction, dans lequel le modèle d'apprentissage automatique prédéfini est configuré pour prédire, au moins en fonction des données de gaz, un stade de cuisson actuel et une probabilité de se trouver dans le stade de cuisson ; et
- la détermination (S103), en fonction du résultat de prédiction, s'il se trouve actuellement dans un stade de cuisson excessive ;
dans lequel la valeur candidate est sélectionnée à partir de l'ensemble de températures de travail prédéfini dans un ordre aléatoire.

2. Procédé de détection de cuisson excessive selon la revendication 1, **caractérisé en ce que** le détecteur de gaz comprend un capteur de composés organiques volatils (COV).

3. Procédé de détection de cuisson excessive selon la revendication 2, **caractérisé en ce que** la valeur candidate est sélectionnée à partir d'un seul ensemble de températures de travail prédéfini comme la température de travail du capteur de composés organiques volatils (COV).

4. Procédé de détection de cuisson excessive selon l'une des revendications 1 à 3, comprenant en outre :
- l'envoi, par un module de commande (12) d'un appareil électroménager (1), d'une instruction de commande lorsqu'il est détecté qu'il se trouve actuellement dans un stade de cuisson excessive ; et
- l'ajustement, par un module d'ajustement (13) de l'appareil électroménager (1), d'un paramètre de cuisson en fonction de l'instruction de commande en réponse à la réception de l'instruction de commande par un module d'ajustement (13).

5. Procédé de détection de cuisson excessive selon l'une des revendications précédentes, **caractérisé en ce que** l'ensemble de températures de travail prédéfini est parcouru pendant au moins un cycle pendant le fonctionnement du détecteur de gaz.

6. Procédé de détection de cuisson excessive selon l'une des revendications précédentes, **caractérisé en ce que** les données de gaz sont générées sur la base de toutes les données de gaz candidates qui sont reçues après que l'ensemble de températures de travail prédéfini est parcouru pour au moins un cycle, ou une quantité de données de gaz candidates pour la génération des données de gaz est inférieure à une quantité des valeurs candidates dans l'ensemble de températures de travail prédéfini.

7. Procédé de détection de cuisson excessive selon l'une des revendications précédentes, **caractérisé en ce que** les valeurs candidates dans l'ensemble de températures de travail prédéfini sont déterminées en fonction de la sensibilité du détecteur de gaz aux composants de gaz à détecter lorsque le détecteur de gaz fonctionne aux valeurs candidates.

8. Procédé de détection de cuisson excessive selon l'une des revendications précédentes, **caractérisé en ce qu'**un processus d'apprentissage du modèle d'apprentissage automatique prédéfini comprend :
- l'obtention (S301) d'un ensemble d'apprentissage, dans lequel l'ensemble d'apprentissage comprend des données de gaz obtenues, respectivement, lorsqu'au moins un ensemble d'aliments se trouve dans une pluralité de stades de cuisson, dans lequel chaque ensemble d'aliments comprend au moins un type d'aliment, et la pluralité de stades de cuisson comprend au moins un stade de cuisson normal et un stade de cuisson excessive ; et
- l'apprentissage (S302) du modèle d'apprentissage automatique prédéfini sur la base de l'ensemble d'apprentissage jusqu'à ce que la précision de prédiction du modèle d'apprentissage automatique prédéfini atteigne un seuil prédéfini.

9. Procédé de détection de cuisson excessive selon la revendication 8, **caractérisé en ce que** le modèle d'apprentissage automatique prédéfini est sélectionné parmi une pluralité de modèles candidats, et **en ce que** le processus d'apprentissage du modèle d'apprentissage automatique prédéfini sélectionné parmi la pluralité de modèles candidats comprend en outre :
- la sélection, parmi une pluralité de modèles candidats obtenus par apprentissage en tant que modèle d'apprentissage automatique prédéfini, avec des modèles candidats différents étant construits par des algorithmes différents, d'un modèle candidat présentant la précision de prédiction la plus élevée en tant que modèle d'apprentissage automatique prédéfini.

10. Procédé de détection de cuisson excessive selon l'une des revendications précédentes, **caractérisé en ce que** l'ensemble d'apprentissage comprend en outre des données de cuisson lorsque chaque donnée de gaz est obtenue, dans lequel les données de cuisson comprennent une température de cuisson et une humidité de cuisson ; et l'entrée des données de gaz dans un modèle d'apprentissage automatique prédéfini et l'obtention d'un résultat de prédiction comprend : l'entrée des données de gaz et des données de cuisson obtenues de manière synchrone avec les données de gaz dans le modèle d'apprentissage automatique prédéfini et l'obtention du résultat de prédiction.

11. Procédé de détection de cuisson excessive selon l'une des revendications précédentes, **caractérisé en ce que** le modèle d'apprentissage automatique prédéfini comprend :
- un premier groupe de modèles comprenant une pluralité de premiers modèles candidats, dans lequel chacun des premiers modèles candidats est obtenu par apprentissage sur la base d'un seul ensemble d'apprentissage, l'ensemble d'apprentissage présente une correspondance biunivoque avec un type d'aliment, et l'ensemble d'apprentissage comprend des données de gaz obtenues, respectivement, lorsqu'un aliment d'un type correspondant se trouve dans une pluralité de stades de cuisson ; et
- un deuxième groupe de modèles comprenant au moins un deuxième modèle candidat, dans lequel chacun de l'au moins un deuxième modèle candidat est obtenu par apprentissage sur la base d'une pluralité d'ensembles d'apprentissage.

12. Procédé de détection de cuisson excessive selon l'une des revendications précédentes, **caractérisé en ce que** l'entrée des données de gaz dans un modèle d'apprentissage automatique prédéfini et l'obtention d'un résultat de prédiction comprennent :
- l'entrée (S1021) des données de gaz dans le premier groupe de modèles et l'obtention (S1021) d'une pluralité de premiers résultats de prédiction délivrés en sortie par les premiers modèles candidats ;
- l'entrée (S1023) des données de gaz dans le deuxième groupe de modèles, si un maximum de probabilité et un maximum de probabilité secondaire dans la pluralité de premiers résultats de prédiction sont similaires et appartiennent à des stades de cuisson différents, et l'obtention (S1023) de deuxièmes résultats de prédiction ; et
- la détermination (S1024), en fonction d'un stade de cuisson auquel appartient un maximum de probabilité dans les premiers résultats de prédiction et les deuxièmes résultats de prédiction, s'il se trouve actuellement dans le stade de cuisson excessive.

13. Procédé de détection de cuisson excessive selon l'une des revendications précédentes, **caractérisé en ce qu'**il comprend en outre :
- la mise à jour d'un ensemble d'apprentissage sur la base des données de gaz obtenues et du résultat de prédiction, dans lequel l'ensemble d'apprentissage comprend des données de gaz obtenues, respectivement, lorsqu'au moins un ensemble d'aliments se trouve dans une pluralité de stades de cuisson ; et
- l'apprentissage du modèle d'apprentissage automatique prédéfini à l'aide de l'ensemble d'apprentissage mis à jour, pour obtenir un modèle d'apprentissage automatique prédéfini mis à jour.

14. Appareil électroménager (1), **caractérisé en ce qu'**il comprend :
- un module de commande (12), configuré pour effectuer le procédé de détection de cuisson excessive selon l'une quelconque des revendications 1 à 13, et pour envoyer une instruction de commande lorsqu'il est détecté qu'il se trouve actuellement dans un stade de cuisson excessive ; et
- un module d'ajustement (13), configuré pour ajuster un paramètre de cuisson en fonction de l'instruction de commande en réponse à la réception de l'instruction de commande.

15. Appareil électroménager (1) selon la revendication 14, **caractérisé en ce que** l'appareil électroménager (1) comprend en outre au moins l'une parmi une table de cuisson (11) ou une hotte aspirante (10).

16. Appareil électroménager (1) selon la revendication 14, **caractérisé en ce qu'**il comprend en outre : un détecteur de gaz (14) configuré pour acquérir les données de gaz, dans lequel le détecteur de gaz (14) communique avec le module de commande (12) pour envoyer les données de gaz au module de commande (12).

17. Appareil électroménager (1) selon l'une des revendications 14 à 16, **caractérisé en ce qu'**il comprend en outre : un module de communication (15), dans lequel le module de commande (12) communique avec le détecteur de gaz (14) à travers le module de communication (15), et/ou le module de commande (12) communique avec le module d'ajustement (13) à travers le module de communication (15).
